# EUROPEAN PATENT APPLICATION

(11) **EP 1 460 136 A1**
(43) Date of publication of application: **22.09.2004**
(21) Application number: 04251613.8
(22) Date of filing: 19.03.2004
(51) Int. Cl.: C12Q 1/68

(54) **Methods for detecting the presence of a nucleic acid analyte in a sample**

(30) Priority: 21.03.2003 US 394112
(71) Applicant: Agilent Technologies, Inc., Palo Alto, CA 94306 (US)
(72) Inventor: Leproust, Eric M., Campbell, CA 95008 (US); Amorese, Douglas A., Los Altos, CA 94022 (US); Ke, Winny W., San Jose, CA 95117 (US)
(74) Representative: Tollett, Ian

(57) **Abstract**

Methods of detecting the presence of a nucleic acid analyte in a sample are provided. In the subject methods, a sample suspected of having an analyte is contacted with an array, and any resultant binding complexes on the surface of the array are detected to determine the presence or absence of the analyte in the sample. A feature of the subject methods is that the sample includes at least a first unlabeled nucleic acid made up of at least two types of nucleotide. Also provided are kits for use in practising the subject methods.

## Description

The field of this invention is nucleic acid arrays.

Array assays between surface bound binding agents or probes and target molecules in solution may be used to detect the presence of particular analytes or biopolymers in the solution. The surface-bound probes may be oligonucleotides, peptides, polypeptides, proteins, antibodies or other molecules capable of binding with target biomolecules in the solution. Such binding interactions are the basis for many of the methods and devices used in a variety of different fields, e.g., genomics (in sequencing by hybridization, SNP detection, differential gene expression analysis, identification of novel genes, gene mapping, finger printing, etc.) and proteomics.

One typical array assay method involves biopolymeric probes immobilized in an array on a substrate such as a glass substrate or the like. A solution suspected of containing an analyte or target molecule(s) ("targets") that bind with the attached probes is placed in contact with the bound probes under conditions sufficient to promote binding of targets in the solution to the complementary probes on the substrate to form a binding complex that is bound to the surface of the substrate. The pattern of binding by target molecules to probe features or spots on the substrate produces a pattern, i.e., a binding complex pattern, on the surface of the substrate which is detected. This detection of binding complexes provides desired information about the target biomolecules in the solution.

The binding complexes may be detected by reading or scanning the array with, for example, optical means, although other methods may also be used, as appropriate for the particular assay. For example, laser light may be used to excite fluorescent labels attached to the targets, generating a signal only in those spots on the array that have a labeled target molecule bound to a probe molecule. This pattern may then be digitally scanned for computer analysis. Such patterns can be used to generate data for biological assays such as the identification of drug targets, single-nucleotide polymorphism mapping, monitoring samples from patients to track their response to treatment, assessing the efficacy of new treatments, etc.

There are two main ways of producing nucleic acid arrays in which the immobilized nucleic acids are covalently attached to the substrate surface: via *in situ* synthesis in which the nucleic acid ligand is grown on the surface of the substrate in a step-wise fashion and via deposition of the full ligand, e.g., a presynthesized nucleic acid/polypeptide, cDNA fragment, etc., onto the surface of the array.

Where the *in situ* synthesis approach is employed, conventional phosphoramidite synthesis protocols are typically used. In phosphoramidite synthesis protocols, the 3'-hydroxyl group of an initial 5'-protected nucleoside is first covalently attached to the polymer support, e.g., a planar substrate surface. Synthesis of the nucleic acid then proceeds by deprotection of the 5'-hydroxyl group of the attached nucleoside, followed by coupling of an incoming nucleoside-3'-phosphoramidite to the deprotected 5' hydroxyl group (5'-OR). The resulting phosphite triester is finally oxidized to a phosphotriester to complete the internucleotide bond. The steps of deprotection, coupling and oxidation are repeated until a nucleic acid of the desired length and sequence is obtained. In other embodiments, the synthesis of the oligonucleotides may be performed in the 5' to 3' direction using analogous protocols and phosphoramidite reagents.

It will be apparent that the effectiveness of employing these arrays depends on the precision with which these oligonucleotides can be synthesized on the substrate surface. As with any chemical process, certain factors may cause the yields of specific steps in the synthesis of oligonucleotides to be less than 100%, resulting in unintended or unwanted intermediate species.

Oftentimes *in situ* synthesis is carried-out by way of highly automated methods that employ array fabricating apparatuses such as pulse-jet fluid deposition technology or other analogous technology, e.g., pin spotting technology. In this manner, a series of droplets, each containing one particular type of reactive deoxynucleoside phosphoramidite is sequentially applied to each discrete area or "feature", sometimes referred to as a "spot" of the array by a pulse-jet printhead. The present inventors have realized that, unfortunately, in certain instances, the precision at which successive droplets can be applied to a feature is insufficient to guarantee that each successive droplet is deposited at the precise location to which it is intended, i.e., to ensure that each successive droplet is confined to the intended feature area or that the entire feature will be covered by any particular droplet. The inventors have realized that misregistration of successively applied droplets may lead to significant amounts of undesriable polymers that are unintentionally synthesized along with a desired polymer within each feature, and may, in addition, lead to synthesis of unwanted polymers in regions of the surface of the array substrate adjacent to each feature.

More specifically, during fabrication of *in situ* oligonucleotide arrays, the oligonucleotide synthesis cycle is spatially controlled to initiate synthesis and perform successive couplings at specific locations on the substrate surface. Accordingly, coupling of the phosphoramidites is spatially controlled using pulse-jet fluid deposition technology and the remainder of the steps, e.g., capping, oxidation, etc., is performed in a flow cell. Consequently, during the synthesis of each successive oligonucleotide layer, the solid support is transferred between an XY stage of a spatially controlled reaction module for coupling and a non-spatially controlled reaction module for capping, oxidation, etc. Therefore, spatial registration and alignment is necessary prior to coupling in the spatially controlled reaction module to ensure that the phosphoramidite reagents are delivered at the same locations as the previous reagents. The inventors have found that a shift or misalignment in the XY stage position and/or in the alignment system that controls the alignment of the deposition head of the spatially controlled reaction module results in a misalignment in the location of the delivered droplets of phosphoramidites reagents at different layers of the synthesis. Consequently, a mixture of full length or intended sequences and unintended sequences may be produced.

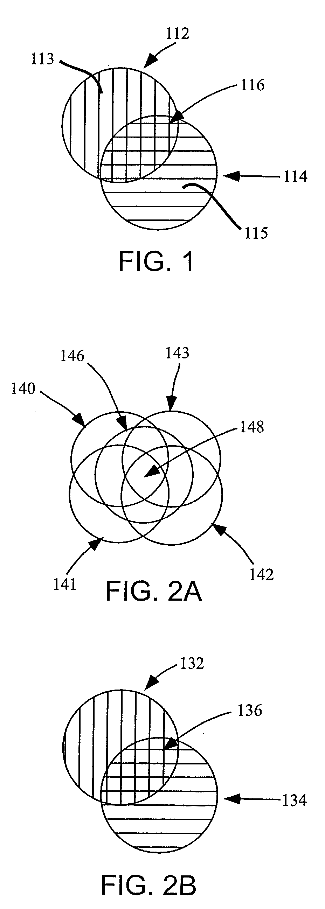

FIG. 1 shows the result of such a misalignment as now understood by the present inventors, where a two step synthesis process, i.e., a two-layer synthesis made of two nucleotides, i.e., a dinucleotide, is illustrated. The misalignment during synthesis due to a shift in the stage and/or in the alignment system results in a first layer or first droplet 112 that includes a first deposited nucleotide and a second layer or second droplet 114 that includes a second deposited nucleotide, where the two layers are not correctly positioned with respect to each other. As shown, due to the misalignment, three discrete regions are produced instead of a desired single region having the full length intended nucleic acid that would have been the result if no misalignment occurred. Accordingly, region 113 is made-up of only the first nucleotide. A misalignment causes the second layer to be shifted with respect to the first layer resulting in a region 115 that is made-up of only the second nucleotide. Due to a portion of the second droplet overlaying a portion of the first droplet, third region 116 is also produced and includes the intended full length sequence made-up of both first and second nucleotides coupled together. This misalignment can be repeated for each sequential nucleotide addition.

Furthermore, the synthesized oligonucleotides may be composed of one or more of, oftentimes all of, four different nucleotides in a particular sequence, where the nucleotides may be delivered by pulse-jet fluid deposition printheads during coupling in the spatially controlled reaction module. Typically, these printheads include one or more nozzles or apertures thereon, through which a precursor reagent, e.g., a particular nucleotide, is dispensed. The precursor reagents are typically contained within one or more reagent reservoirs that are associated with the printheads, and more specifically one or more nozzles of a printhead. The number of nozzles per reservoir or per printhead may vary and may range from about 2 about 1024, e.g., from about 20 to about 256. Accordingly, the number of printheads employed may vary and may range from four printheads such that each type of nucleotide may be deposited by an individual, independent printhead to two printheads such that two types of nucleotides may be delivered by a single printhead having two reagent reservoirs associated therewith.

Thus, the relative misalignment between two printheads may therefore be determined by the relative alignment of the printhead nozzles with respect to each other, e.g., a nozzle of one printhead relative to a nozzle of another printhead. Accordingly, a misalignment of any of these printheads, or rather the nozzles of the printheads, relative to each other will produce a mixture of full length sequences, i.e., intended sequences, and unintended sequences.

Regardless of how the unintended sequences are produced, when contacted with a sample containing labeled target molecules during an array assay, not only can the full length intended sequence bind labeled target molecules in the sample, but also one or more unintended sequences can bind labeled target molecules in the sample.

The inventors have realized that the presence of these undesirable polymers produced on the substrate surface may lead to less specific binding of radioactively, fluorescently or chemiluminescently labeled target to the array, in turn leading to a significant decrease in the signal-to-noise ratio in the analysis of the array which may compromise array assay results.

Accordingly, there continues to be an interest in the development of new methods to eliminate the effects of pulse-jet fluid deposition misalignments. Of particular interest is the development of such methods that are easy to use, are effective at eliminating the results of the misalignments, and which do not add additional steps to a conventional analyte detection protocol.

Methods of detecting the presence of a nucleic acid analyte in a sample are provided. In the subject methods, a sample suspected of having an analyte is contacted with an array, and any resultant binding complexes on the surface of the array are detected to determine the presence or absence of the analyte in the sample. A feature of the subject methods is that the sample includes at least a first unlabeled nucleic acid made up of at least two types of nucleotides. Also provided are kits for use in practicing the subject methods.

Embodiments of the present invention are described below, by way of example only, with reference to the accompanying drawings in which:
Figure 1 shows the effects of misalignment of a fluid deposition device and a location of a substrate surface such as the effects of misalignment of a stage such as an XYZ stage and/or an alignment system of a fluid deposition device all as now understood by the present inventors.
Figure 2A shows the effects of misalignment of all four printheads of a fluid deposition device relative to each other, and Figure 2B shows the effects of misalignment between two printheads of a fluid deposition device relative to each other, wherein each printhead delivers two monomers different from the two monomer delivered from the other printhead and any other misalignments (e.g., of the alignment system, stage errors, etc.) are assumed to be negligible.
Figure 3A schematically illustrates a feature of an array having two "crescent" areas of unintended sequences, as well as a third area of the intended full length sequence.
Figure 3B shows a side-view of the feature of an array of Figure 3A.
Figure 3C shows a step of the subject methods wherein a sample containing at a first unlabeled nucleic acid and a second unlabeled nucleic acid sequence is contacted to the feature of Figures 3A and 3B such that the unlabeled nucleic acids form binding complexes with complementary unintended sequences present in the crescent areas of the array.
Figure 3D illustrates a step of the subject methods wherein unlabeled nucleic acid in a sample effectively competes with labeled analyte in the sample to prevent labeled analyte from binding to unintended sequences present in the crescent areas of the array.
Figure 4 illustrates a step of the subject methods wherein a sample containing labeled nucleic acid analyte complementary to the intended array sequences and also includes only unlabeled nucleic acid made up of two nucleotides and having a random sequence is contacted with an array to prevent labeled analyte from binding to unintended sequences present in the crescent areas of the feature.
Figures 5A and 5B show the results of contacting a substrate having areas of unintentionally synthesized sequences as intentionally synthesized full-length sequences with a sample not having any unlabeled nucleic acid sequences (Figure 5A) and a sample having unlabeled nucleic acid sequences (Figure 5B).

The term "nucleic acid" as used herein means a polymer composed of nucleotides, e.g. deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in hybridization reactions, i.e., cooperative interactions through Pi electrons stacking and hydrogen bonds, such as Watson-Crick base pairing interactions, Wobble interactions, etc.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length.

The term "polynucleotide" as used herein refers to single or double stranded polymer composed of nucleotide monomers of generally greater than 100 nucleotides in length.

The term "monomer" as used herein refers to a chemical entity that can be covalently linked to one or more other such entities to form an oligomer. Examples of "monomers" include nucleotides, amino acids, saccharides, peptides, and the like. In general, the monomers used in conjunction with the present invention have first and second sites (e.g., C-termini and N-termini, or 5' and 3' sites) suitable for binding to other like monomers by means of standard chemical reactions (e.g., condensation, nucleophilic displacement of a leaving group, or the like), and a diverse element which distinguishes a particular monomer from a different monomer of the same type (e.g., an amino acid side chain, a nucleotide base, etc.). The initial substrate-bound monomer is generally used as a building-block in a multistep synthesis procedure to form a complete ligand, such as in the synthesis of oligonucleotides, oligopeptides, and the like.

The term "oligomer" is used herein to indicate a chemical entity that contains a plurality of monomers. As used herein, the terms "oligomer" and "polymer" are used interchangeably. Examples of oligomers and polymers include polydeoxyribonucleotides (DNA), polyribonucleotides (RNA), other polynucleotides which are C-glycosides of a purine or pyrimidine base, polypeptides (proteins), polysaccharides (starches, or polysugars), and other chemical entities that contain repeating units of like chemical structure.

The terms "nucleoside" and "nucleotide" are intended to include those moieties which contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

"Optional" or "optionally" means that the subsequently described circumstance may or may not occur, so that the description includes instances where the circumstance occurs and instances where it does not. For example, the phrase "optionally capping" means that a capping step may or may not be performed, and, thus, the description includes embodiments wherein a capping step is performed and embodiments wherein a capping step is not performed.

An "array," includes any one, two-dimensional or substantially two-dimensional (as well as a three-dimensional) arrangement of addressable regions bearing a particular chemical moiety or moieties (e.g., biopolymers such as polynucleotide or oligonucleotide sequences (nucleic acids), polypeptides (e.g., proteins), carbohydrates, lipids, etc.) associated with that region. In the broadest sense, the preferred arrays are arrays of polymeric binding agents, where the polymeric binding agents may be any of: polypeptides, proteins, nucleic acids, polysaccharides, synthetic mimetics of such biopolymeric binding agents, etc. In many embodiments of interest, the arrays are arrays of nucleic acids, including oligonucleotides, polynucleotides, cDNAs, mRNAs, synthetic mimetics thereof, and the like. Where the arrays are arrays of nucleic acids, the nucleic acids may be covalently attached to the arrays at any point along the nucleic acid chain, but are generally attached at one of their termini (e.g. the 3' or 5' terminus). Sometimes, the arrays are arrays of polypeptides, e.g., proteins or fragments thereof.

Any given substrate may carry one, two, four or more or more arrays disposed on a front surface of the substrate. Depending upon the use, any or all of the arrays may be the same or different from one another and each may contain multiple spots or features. A typical array may contain more than ten, more than one hundred, more than one thousand more ten thousand features, or even more than one hundred thousand features, in an area of less than 20 cm2 or even less than 10 cm2. For example, features may have widths (that is, diameter, for a round spot) in the range from a 10 µm to 1.0 cm. In other embodiments each feature may have a width in the range of 1.0 µm to 1.0 mm, usually 5.0 µm to 500 µm, and more usually 10 µm to 200 µm. Non-round features may have area ranges equivalent to that of circular features with the foregoing width (diameter) ranges. At least some, or all, of the features are of different compositions (for example, when any repeats of each feature composition are excluded the remaining features may account for at least 5%, 10%, or 20% of the total number of features). Interfeature areas will typically (but not essentially) be present which do not carry any polynucleotide (or other biopolymer or chemical moiety of a type of which the features are composed). Such interfeature areas typically will be present where the arrays are formed by processes involving drop deposition of reagents but may not be present when, for example, photolithographic array fabrication processes are used. It will be appreciated though, that the interfeature areas, when present, could be of various sizes and configurations.

Each array may cover an area of less than 100 cm², or even less than 50 cm², 10 cm² or 1 cm². In many embodiments, the substrate carrying the one or more arrays will be shaped generally as a rectangular solid (although other shapes are possible), having a length of more than 4 mm and less than 1 m, usually more than 4 mm and less than 600 mm, more usually less than 400 mm; a width of more than 4 mm and less than 1 m, usually less than 500 mm and more usually less than 400 mm; and a thickness of more than 0.01 mm and less than 5.0 mm, usually more than 0.1 mm and less than 2 mm and more usually more than 0.2 and less than 1 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, a substrate may transmit at least 20%, or 50% (or even at least 70%, 90%, or 95%), of the illuminating light incident on the front as may be measured across the entire integrated spectrum of such illuminating light or alternatively at 532 nm or 633 nm.

An array is "addressable" when it has multiple regions of different moieties (e.g., different polynucleotide sequences) such that a region (i.e., a "feature" or "spot" of the array) at a particular predetermined location (i.e., an "address") on the array will detect a particular target or class of targets (although a feature may incidentally detect non-targets of that feature). Array features are typically, but need not be, separated by intervening spaces. In the case of an array, the "target" will be referenced as a moiety in a mobile phase (typically fluid), to be detected by probes ("target probes") which are bound to the substrate at the various regions. However, either of the "target" or "target probe" may be the one which is to be evaluated by the other (thus, either one could be an unknown mixture of polynucleotides to be evaluated by binding with the other). A "scan region" refers to a contiguous (preferably, rectangular) area in which the array spots or features of interest, as defined above, are found. The scan region is that portion of the total area illuminated from which the resulting fluorescence is detected and recorded. For the purposes of this invention, the scan region includes the entire area of the slide scanned in each pass of the lens, between the first feature of interest, and the last feature of interest, even if there exists intervening areas which lack features of interest. An "array layout" refers to one or more characteristics of the features, such as feature positioning on the substrate, one or more feature dimensions, and an indication of a moiety at a given location. "Hybridizing" and "binding", with respect to polynucleotides, are used interchangeably.

"Remote location," means a location other than the location at which the array is present and hybridization occurs. For example, a remote location could be another location (e.g., office, lab, etc.) in the same city, another location in a different city, another location in a different state, another location in a different country, etc. As such, when one item is indicated as being "remote" from another, what is meant is that the two items are at least in different rooms or different buildings, and may be at least one mile, ten miles, or at least one hundred miles apart.

"Communicating" information references transmitting the data representing that information as electrical signals over a suitable communication channel (e.g., a private or public network).

"Forwarding" an item refers to any means of getting that item from one location to the next, whether by physically transporting that item or otherwise (where that is possible) and includes, at least in the case of data, physically transporting a medium carrying the data or communicating the data.

Methods of detecting the presence of a nucleic acid analyte in a sample are provided. In the subject methods, a sample suspected of having an analyte is contacted with an array, and any resultant binding complexes on the surface of the array are detected to determine the presence or absence of the analyte in the sample. A feature of the subject methods is that the sample includes at least a first unlabeled nucleic acid made up of at least two types of nucleotides. Also provided are kits for use in practicing the subject methods.

Before the present invention is described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "an", and "the" include plural referents unless the context clearly dictates otherwise.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope of the present invention.

The figures shown herein are not necessarily drawn to scale, with some components and features being exaggerated for clarity.

As summarized above, the subject invention provides methods of detecting the presence of a nucleic acid analyte in a sample. In further describing the subject methods, a review of representative arrays that may be used with the subject methods will be described first to provide a proper foundation for describing the invention. Next, the subject methods will be described in detail, followed by a description of kits for use with the subject methods.

### REPRESENTATIVE ARRAYS

As mentioned above, the methods of the subject invention are directed to detecting the presence of a nucleic acid analyte in a sample using an array. Such arrays find use in a variety of applications, including gene expression analysis, drug screening, nucleic acid sequencing, mutation analysis, and the like. These arrays include a plurality of ligands or molecules or probes (i.e., binding agents or members of a binding pair) deposited onto the surface of a substrate in the form of an "array" or pattern.

The subject arrays include at least two distinct polymers that differ by monomeric sequence attached to different and known locations on the substrate surface. Each distinct polymeric sequence of the array is typically present as a composition of multiple copies of the polymer on a substrate surface, e.g., as a spot or feature on the surface of the substrate. The number of distinct polymeric sequences, and hence spots or similar structures, present on the array may vary, where a typical array may contain more than about ten, more than about one hundred, more than about one thousand, more than about ten thousand or even more than about one hundred thousand features in an area of less than about 20 cm² or even less than about 10 cm². For example, features may have widths (that is, diameter, for a round spot) in the range from about 10 µm to about 1.0 cm. In other embodiments, each feature may have a width in the range from about 1.0 µm to about 1.0 mm, usually from about 5.0 µm to about 500 µm and more usually from about 10 µm to about 200 µm. Non-round features may have area ranges equivalent to that of circular features with the foregoing width (diameter) ranges. At least some, or all, of the features are of different compositions (for example, when any repeats of each feature composition are excluded, the remaining features may account for at least about 5%, 10% or 20% of the total number of features). Interfeature areas will typically (but not essentially) be present which do not carry any polynucleotide (or other biopolymer or chemical moiety of a type of which the features are composed). Such interfeature areas typically will be present where the arrays are formed by processes involving drop deposition of reagents, but may not be present when, for example, photolithographic array fabrication process are used. It will be appreciated though, that the interfeature areas, when present, could be of various sizes and configurations. The spots or features of distinct polymers present on the array surface are generally present as a pattern, where the pattern may be in the form of organized rows and columns of spots, e.g. a grid of spots, across the substrate surface, a series of curvilinear rows across the substrate surface, e.g. a series of concentric circles or semi-circles of spots, and the like.

In the broadest sense, the arrays are arrays of polymeric or biopolymeric ligands or molecules, i.e., binding agents, where the polymeric binding agents are nucleic acids and synthetic mimetics thereof. In many embodiments of interest, the arrays are arrays of nucleic acids, including oligonucleotides, polynucleotides, cDNAs, mRNAs, synthetic mimetics thereof, and the like.

The arrays may be produced using any convenient protocol. One such method involves the *in situ* fabrication of arrays using drop deposition of reagents from pulse jets of polynucleotide precursor units (such as monomers). Such methods are described in detail in, for example, the previously cited references including U.S. Patent Nos.: 6,242,266, 6,232,072, 6,180,351, 6,171,797, and 6,323,043, and U.S. Patent Application Serial No. 09/302,898 filed April 30, 1999 by Caren et al., and the references cited therein, the disclosures of which are herein incorporated by reference. Other deposition methods using pulse jet technology may be used for fabrication.

A variety of solid supports or substrates may be used, upon which an array may be positioned. In certain embodiments, a plurality of arrays may be stably associated with one substrate. For example, a plurality of arrays may be stably associated with one substrate, where the arrays are spatially separated from some or all of the other arrays associated with the substrate.

The array substrate may be selected from a wide variety of materials including, but not limited to, natural polymeric materials, particularly cellulosic materials and materials derived from cellulose, such as fiber containing papers, e.g., filter paper, chromatographic paper, etc., synthetic or modified naturally occurring polymers, such as nitrocellulose, cellulose acetate, poly (vinyl chloride), polyamides, polyacrylamide, polyacrylate, polymethacrylate, polyesters, polyolefins, polyethylene, polytetrafluoro-ethylene, polypropylene, poly (4-methylbutene), polystyrene, poly(ethylene terephthalate), nylon, poly(vinyl butyrate), cross linked dextran, agarose, etc.; either used by themselves or in conjunction with other materials; fused silica (e.g., glass), bioglass, silicon chips, ceramics, metals, and the like. For example, substrates may include polystyrene, to which short oligophosphodiesters, e.g., oligonucleotides ranging from about 5 to about 50 nucleotides in length, may readily be covalently attached (Letsinger et al. (1975) *Nucl. Acids Res.* 2:773-786), as well as polyacrylamide (Gait et al. (1982) *Nucl. Acids Res.* 10:6243-6254), silica (Caruthers et al. (1980) *Tetrahedron Letters* 21:719-722), and controlled-pore glass (Sproat et al. (1983) *Tetrahedron Letters* 24:5771-5774). Additionally, the substrate can be hydrophilic or capable of being rendered hydrophilic.

Suitable array substrates may exist, for example, as sheets, tubing, spheres, containers, pads, slices, films, plates, slides, strips, disks, etc. The substrate is usually flat, but may take on alternative surface configurations. The substrate can be a flat glass substrate, such as a conventional microscope glass slide, a cover slip and the like. Common substrates used for the arrays of probes are surface-derivatized glass or silica, or polymer membrane surfaces, as described in Maskos, U. et al., *Nucleic Acids Res,* 1992, 20:1679-84 and Southern, E. M. et al., *Nucleic acids Res,* 1994, 22:1368-73.

Each array may cover an area of less than about 100 cm², or even less than about 50 cm², 10 cm² or 1 cm². In many embodiments, the substrate carrying the one or more arrays will be shaped generally as a rectangular solid (although other shapes are possible), having a length of more than about 4 mm and less than about 1 m, usually more than about 4 mm and less than about 600 mm, more usually less than about 400 mm; a width of more than about 4 mm and less than about 1 m, usually less than about 500 mm and more usually less than about 400 mm; and a thickness of more than about 0.01 mm and less than about 5.0 mm, usually more than about 0.1 mm and less than about 2 mm and more usually more than about 0.2 and less than about 1 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, the substrate may transmit at least about 20%, or about 50% (or even at least about 70%, 90%, or 95%), of the illuminating light incident on the substrate as may be measured across the entire integrated spectrum of such illuminating light or alternatively at 532 nm or 633 nm.

As mentioned above, an array contains multiple spots or features of biopolymers, e.g., in the form of oligonucleotides, polynucleotides or the like. As mentioned above, all of the features may be different, or some or all could be the same. The interfeature areas, if present, could be of various sizes and configurations. Each feature carries a predetermined biopolymer such as a predetermined polynucleotide (which includes the possibility of mixtures of oligonucleotides). It will be understood that there may be a linker molecule of any known types between the substrate surface and the first nucleotide.

### METHODS OF DETECTING THE PRESENCE OF ANALYTE IN A SAMPLE

As summarized above, the subject invention provides methods for detecting the presence of a nucleic acid analyte in a sample. More specifically, the subject methods include the steps of: (a) contacting a nucleic acid array with a sample suspected of including an analyte, wherein the sample includes at least a first unlabeled nucleic acid made up of only two types of nucleotides and having a random sequence; (b) detecting any binding complexes on the surface of the array to obtain binding complex data; and (c) determining the presence of the analyte in the sample using the binding complex data.

FIG. 2A illustrates the effect of a relative misalignment with respect to the intended position 146, between four independent printheads, each of which deposits one type of monomer or phosphoramidite reagent. As illustrated, first through fourth drops of reagents 140, 141, 142 and 143 are misaligned. In this example, misalignment occurs at every layer, i.e., each coupling step, such that only the region of commonality between the droplets, herein represented by reference numeral 148, contains the full length intended nucleic acid sequence while four unintended sequences are also produced adjacent thereto. It will be apparent that in practice, all combinations of nucleic acid sequences may be produced due to the misalignment, i.e., A, AT, ATC, ATCG, ATG, AC, ACG, AG, T, TC, TCG, TG, C, CG and G. In many instances, two types ofphosphoramidites are delivered from a single printhead, as mentioned above. Accordingly, in this case the two phosphoramidites are aligned with respect to each other within the same printhead, but misaligned with respect to the other printhead or rather the other two phosphoramidites delivered from a second printhead. For example, G and T phosphoramidites may be delivered from a first printhead and C and A phosphoramidites may be delivered from a second printhead. Thus, G and T are aligned with respect to each other, but misaligned with respect to C and A. FIG. 2B illustrates the result of such misalignment. As shown, three regions are synthesized: a central region 136 or feature containing the intended full length sequence containing all four intended nucleotides A, C, T and G, and two "crescent" regions of unintended sequences containing sequences of G and T only (region 132) and sequences of A and T only (region 134) such that regions 132 and 134 that re adjacent feature 136 have incomplete sequences. It will be appreciated that in certain embodiments each printhead may deposit all four phosphoramidites such that two, three or four phosphoramidites may be misaligned relative to each other such that the phosphoramidites deposited from any one given printhead are aligned with respect to each other, but may be misaligned with respect to any one or more phosphoramidites deposited from any other printhead.

In order to automatically scan or read arrays for the presence of radioactively, fluorescently or chemiluminescently labeled targets, it is most desirable for the surfaces of the features to be uniformly covered with desired surface-bound polymers, and for each feature to have a sharply defined edge. The inter-feature areas of the array should have little or no contaminants that can bind the targets, including substrate-bound polymers inadvertently synthesized along with the intended polymers synthesized within the features. Otherwise, after exposure of the array to labeled sample molecules, fuzzy, indiscrete area of the array substrate will contain labeled target molecules, making it difficult for the software used to analyze the features to select an area for signal intensity averaging. Poorly averaged signal intensity may significantly lower confidence in resulting measurements, and may even produce incorrect results.

Accordingly, a nucleic acid array such as that described above, is provided and contacted with a sample suspected of having an analyte, i.e., suspected of including one or more target nucleic acids. Target nucleic acids in the sample are typically prepared with a label, e.g., a member of signal producing system, so that when bound to the array to form a binding complex with a complementary nucleic acid bound to the array's substrate, the target nucleic acids may be detected. Accordingly, if the sample includes a target analyte, the sample thus includes labeled target nucleic acid and at least one unlabeled nucleic acid.

Thus, at some point prior to the detection step described below, any target analyte present in the initial sample contacted with the array is labeled with a detectable label. Labeling may occur either prior to or following contact with the array. In other words, the analyte, e.g., nucleic acids, present in the fluid sample contacted with the array may be labeled prior to or after contact, e.g., hybridization, with the array. In some embodiments of the subject methods, the sample analytes e.g., target nucleic acids, are directly labeled with a detectable label, wherein the label may be covalently or non-covalently attached to the nucleic acids of the sample. For example, the nucleic acids, including the target nucleotide sequence, may be labeled with biotin, exposed to binding conditions, wherein the labeled target nucleotide sequence binds to an avidin-label or an avidin-generating species. In an alternative embodiment, the target analyte such as the target nucleotide sequence is indirectly labeled with a detectable label, wherein the label may be covalently or non-covalently attached to the target nucleotide sequence. For example, the label may be non-covalently attached to a linker group, which in turn is (i) covalently attached to the target nucleotide sequence, or (ii) comprises a sequence which is complementary to the target nucleotide sequence. In another example, the probes may be extended, after hybridization, using chain-extension technology or sandwich-assay technology to generate a detectable signal (see, e.g., U.S. Patent No. 5,200,314). Generally, such detectable labels include, but are not limited to, radioactive isotopes, fluorescers, chemiluminescers, enzymes, enzyme substrates, enzyme cofactors, enzyme inhibitors, dyes, metal ions, ligands (e.g., biotin or haptens) and the like.

In certain embodiments, the label is a fluorescent compound, i.e., is capable of emitting radiation (visible or invisible) upon stimulation by radiation of a wavelength different from that of the emitted radiation, or through other manners of excitation, e.g., chemical or non-radiative energy transfer. The label may be a fluorescent dye. Usually, a target with a fluorescent label includes a fluorescent group covalently attached to a nucleic acid molecule capable of binding specifically to the complementary probe nucleotide sequence.

A feature of the subject methods is that the sample suspected of including an analyte and which is contacted with the array also includes at least a first unlabeled nucleic acid made up of at least two types of nucleotides i.e., at least two different nucleotides, oftentimes just two types of nucleotides (oftentimes just one type of nucleotide as will be described below), and having a random sequence. That is, the sample includes at least one random nucleic acid sequence made up of at least two different types of unlabeled nucleotides or binding mimetics thereof. In other words, the sample contains an unlabeled sequence that is made up of at least two of the four different types of nucleotides representing the four different bases, i.e., A, G, C and T, such that the sample contains at least a first unlabeled nucleic acid sequence made up of at least two of: A, G, C and T, e.g., may be made of just two, three or all four of: A, G, C and T. For example, a subject unlabeled nucleic acid sequence may be made up of a random sequence of only: T and G, C and A, T and C, A and G, G and C, or G and A. By random sequence it is meant that the subject unlabeled nucleic acid sequence or plurality of sequences are not specifically chosen to hybridize to a particular nucleic acid sequence or probe of an array, such that prior to contact with the array it is not known whether the unlabeled nucleic acid(s) will hybridize under appropriate conditions to any of the bound nucleic acids and each unlabeled nucleic acid. Randomness may include all possible permutations of the nucleotides, or may include a certain degree of randomness such that less than all possible permutations are present, where such a degree may range from 99% of all possible permutations to 5% or less than 5% of all possible permutations, e.g., may be as little as about 1% in certain instances. By "unlabeled" it is meant that the nucleic acids employed to bind to the unintentionally synthesized sequences adjacent a feature of an array may not have a label at all, or may include a label as long as the label is distinguishable from a target label, i.e., distinguishable from a label used to identify bound target (i.e., binding complexes on the surface of the array) by whatever protocol is employed to identify bound target. For example, if a given target is labeled with a particular fluorescent label, the at least one unlabeled nucleic acid of the subject invention may be devoid of any label, or may have a label that is not the same fluorescent label used with the target such that, e.g., it may fluoresce at a different wavelength or may not be a fluorescent label at all, e.g., it may be a radioactive label or the like.

As will be described below, a given nucleic acid sequence may also be a homopolymer, e.g., be made up of all A, all T, all C or all G, such that a given nucleic acid sequence may be made of only one type of nucleotide. In many embodiments a population of heteropolymers and homopolymers may be employed, i.e., a mixture of at least one hetero- and at least one homopolymer. In many embodiments, a plurality of heteropolymers (e.g., different permutations or combination of two nucleotides) and a plurality of homopolymers may be used. For example in regards to the homopolymers, for any two nucleotide combination, such as the same two that make up the heteropolymers, each nucleotide thereof may be present as a homopolymer and may be present as multiple homopolymers of various lengths.

The subject invention also includes tailoring or selecting one or more unlabeled nucleic acids for a particular protocol such that the subject invention includes methods for determining one or more unlabeled nucleic acid sequences, which should be used with an *in situ* synthesized nucleic acid array, to preferentially bind incomplete sequences adjacent one or more array features. For example, the subject invention includes employing a particular unlabeled nucleic acid sequence in a given protocol by determining the nucleic acid sequence(s) that have been unintentionally synthesized (i.e., the incomplete sequences adjacent an array feature (the crescent shaped regions) due to misalignment. In other words, a misalignment between different nucleotides deposited onto a surface by an array fabricating device may be determined. Once a misalignment between different nucleotides deposited onto a surface by an array fabricating device is determined, the subject methods also include determining, as the unlabeled nucleic acid, at least a first unlabeled nucleic acid made up of nucleotides the selection of which is based on the nucleotides determined to be misaligned, and having a random sequence.

The length of an unlabeled nucleic acid present in a sample may vary depending on a variety of factors such as the amount of unlabeled nucleic acid in the sample, the particular array with which the sample is contacted, the amount and length of the labeled target employed, etc. Typically, the length of an unlabeled nucleic acid in a sample ranges from about 5 mers to about 40 mers or more, usually from about 6 mers to about 20 mers and more usually from about 8 mers to about 15 mers. In those embodiments having more than one unlabeled nucleic acid in a sample, the lengths of the mixture of unlabeled nucleic acids present may vary such that there may be a variety of different sizes of nucleic acids present in the sample. That is, all of the unlabeled nucleic acids present may have the same lengths or some or all of the unlabeled nucleic acid may have different lengths. In such embodiments, the average length of all of the members of the set of unlabeled nucleic acids will typically fall within the above ranges.

Typically, each unlabeled nucleic acid sequence is present as multiple copies in the sample. The amount or concentration of a particular unlabeled nucleic acid in a sample may depend on a variety of factors such as the size of the particular nucleic acid, the number and amount of other unlabeled nucleic acids present, the particular array with which the sample is used, etc. Typically, an unlabeled nucleic acid having a size from about 5 mers to about 40 mers will be present in a sample in an amount ranging from about 0.001 mg/ml to about 1 mg/ml, usually from about 0.001 mg/ml to about 0.1 mg/ml and more usually from about 0.01 mg/ml to about 0.1 mg/ml.

In many embodiments, the sample may include a population or mixture of different unlabeled nucleic acids, where the different nucleic acids, i.e., sub-populations, may differ in one or more respects such as in sequence and/or size and/or nucleotides employed to make up the sequence, etc., as will be described below. The different unlabeled nucleic acids may be present in substantially proportionate amounts, e.g., present in substantially equimolar, including equimolar amounts, in the sample or may be present in varying amounts in the sample. Where there is a difference in the relative amounts of each unlabeled nucleic acid present in a sample, the difference generally does not exceed about 1000 fold and usually does not exceed about 100 fold.

For example, certain embodiments may include more than one nucleic acid sequence such that two or more different nucleic acid sequences may be present in a given sample. Accordingly, in those embodiments having two or more different unlabeled nucleic acids, the nucleic acids may differ in the sequence of nucleotides such that each of the members of such a population may be made of the same nucleotides, however each member may have a unique random sequence. In other words, all the unlabeled nucleic acids may be made up of the same one, two, three or all four nucleotides, but each nucleic acid may differ from the other nucleic acids in that each nucleic acid has a unique random sequence. For such a population of random sequences, all unique random sequences may be present in the population i.e., all permutations of possible sequences of the nucleotides may be present, and some or all unique random sequences may be present as multiple copies. For example, in a population of, for example, 10mer oligos made of A and C, all permutations of A and C may be employed, including homopolymers of each, e.g., AAAAAAAAAA and CCCCCCCCC. Analogously, in a population of, for example, 10mer oligos made of A, C and T, homopolymers of each may be employed, e.g., AAAAAAAAAA, CCCCCCCCCC and TTTTTTTTTT. Such populations that include all such permutations of the nucleotides of interest, e.g., populations that include homopolymers and heteropolymers of the nucleotides, may be characterized as a random distribution of formula AₓC_{y} where x=0 up to the maximum number of nucleotides of a sequence, e.g., 10 for a 10 mer oligonucleotide ("oligo") such that for a 10 mer oligo x=0 to 10, and y=0 up to the maximum number of nucleotides of a sequence, e.g., 10 for a 10 mer oligo such that for a 10 mer oligo y=0 to 10 and x+y = 10 (for a 10 mer oligo). In many embodiments, "x" and "y" may be as high as about 40 mer or more. The above-described formula is of course applicable to any nucleotides such as AₓT_{y}, AₓG_{y}, CₓT_{y}, CₓG_{y}, TₓG_{y}, etc.

Each nucleic acid of the population may be made up of only the same at least two nucleotides as the other unlabeled nucleic acids in the population, e.g., made up of only: A and C, or A and T, or A and G, or C and T, or C and G, or G and T, but has a unique random sequence such that the sequence of nucleotides varies amongst the different nucleic acids such that no two unlabeled nucleic acids have the same nucleotide sequence, i.e., each has a unique sequence, usually present in multiple copies. The members of this population all may have the same length or some or all may have different lengths and this population may include homopolymers as well.

Any two sequences are considered different for purposes of the present invention if there is as little as one base variation in terms of length and/or base composition, upon alignment of the sequences. Sequence similarity can be determined in a number of different manners. To determine sequence identity, sequences can be aligned using the methods and computer programs, including BLAST, available over the world wide web at ncbi.nlm.nih.gov/BLAST/. Another alignment algorithm is FASTA, available in the Genetics Computing Group (GCG) package, from Madison, Wisconsin, USA, a wholly owned subsidiary of Oxford Molecular Group, Inc. Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Of particular interest are alignment programs that permit gaps in the sequence. The Smith-Waterman is one type of algorithm that permits gaps in sequence alignments. See *Meth. Mol. Biol.* 70: 173-187 (1997). Also, the GAP program using the Needleman and Wunsch alignment method can be utilized to align sequences. See *J. Mol. Biol.* 48: 443-453 (1970)

One particular method that may be employed utilizes the BestFit program using the local homology algorithm of Smith Waterman (Advances in Applied Mathematics 2: 482-489 (1981) to determine sequence identity. The gap generation penalty will generally range from 1 to 5, usually 2 to 4 and in many embodiments will be 3. The gap extension penalty will generally range from about 0.01 to 0.20 and in many instances will be 0.10. The program has default parameters determined by the sequences inputted to be compared. Preferably, the sequence identity is determined using the default parameters determined by the program. This program is available also from Genetics Computing Group (GCG) package, from Madison, Wisconsin, USA.

The number of different unlabeled nucleic acid sequences that are made up of only the same nucleotides, but which nucleic acids differ only in sequence (e.g., a population of only A and C, only G and T, etc.), i.e., the number of sub-populations or number of different sequences, in a sample will vary, depending on a variety of factors such as the lengths of the nucleic acids, the number of nucleotides employed to make up the nucleic acids, etc., but will typically range from about 2 to about 2⁴⁰, usually from about 2⁶ to about 2²⁰ and more usually from about 2⁸ to about 2¹⁴.

The amount of each constituent member of this population of nucleic acids made of the same nucleotides but which differ in the sequence of the nucleotides or amount of each sub-population present in the sample may be the substantially the same as the amount of any or all of the other members, i.e., substantially proportionate or equimolar, including equimolar, or may differ such that one or more unlabeled unique random nucleic acid sequences may be present in a sample in a greater amount than one or more other unlabeled sequence(s), i.e., the different unlabeled nucleic acids may be present in a substantially disproportionate amounts, where the amount or proportion of each constituent member of this population may depend at least on the size of the unlabeled sequences, the number of different random nucleic acid sequences present, etc. Where there is a difference in the relative amounts of each unlabeled nucleic acid present in a sample, the difference generally does not exceed about 1000 fold and usually does not exceed about 100 fold.

In certain embodiments, the sample may include two or more different unlabeled nucleic acids or mixtures of at least two different unlabeled nucleic acids, where the nucleic acids differ from one another in the type of nucleotides that make up the different nucleic acids, and each nucleic acid has a random sequence, e.g., a unique random sequence. In many embodiments, a sample may include at least two populations of different nucleic acids such that each population includes a plurality of nucleic acids -each having a unique random sequence, and each may be present in multiple copies. For example, a first nucleic acid may be made up of a nucleic acid sequence made of at least two types of nucleotide (e.g., two of A, C, T or G), or a first population may include a plurality of nucleic acid members-each made up of only the at least two types of nucleotides, but which members differ in sequence and/or length. A second nucleic acid may be made up of a nucleic acid sequence made of at least two different types of nucleotide than the first nucleic acid (e.g., two of A, C, T or G), or a second population may include a plurality of nucleic acid members-each made up of only the at least two types of nucleotides, but which members differ in sequence and/or length. Accordingly, the first population may differ from the second population in that the nucleic acid(s) of the two populations may be made up of two different nucleotides and each nucleic acid of each population has a unique random sequence.

As the different nucleic acids and respective different nucleic acid populations have been described above with respect to nucleic acid compositions of two types of nucleotides, it will be apparent that the nucleic acids may be made of one type, three or all four types of nucleotides. For example, each different nucleic acid sequence may be made up of three nucleotides, i.e., three of A or C or G or T and have a unique random sequence such that the different nucleic acids differ from each other by one of the nucleotides. For example, a first unlabeled sequence may be made up of A, C and T and a second unlabeled nucleic acid sequence may be made up of A, C and G, where as mentioned above each may be present in multiple copies. Likewise, a first population may be made up of a plurality of nucleic acid sequences such that all the members are made up of A, C and T and each has a unique random sequence and/or some or all of the members have different lengths. Accordingly, a second population may be made up of a plurality of nucleic acid sequences such that all the members are made up of A, C and T and each has a unique random sequence and/or some or all of the members have different lengths.

The amount of each different nucleic acid present in the sample may be the substantially the same, i.e., substantially proportionate or equimolar, including equimolar, or may differ from the amount of another nucleic acid such that, for example, a first unlabeled nucleic acid sequence may be present in a sample in a greater or lesser amount than one or more other unlabeled sequence(s). In other words, the different unlabeled nucleic acids may be present in substantially disproportionate amounts, where the amount or proportion of each constituent member of this population may depend at least on the size of the unlabeled sequences, the number of different random nucleic acid sequences present, etc.

Accordingly, a first population may include at least a first unlabeled nucleic acid sequence that may be characterized by a first formula Kₙ and a second population that may include at least a second unlabeled nucleic acid sequence that may be characterized by a second formula Mₙ, where K is A or C or G or T and M is A or C or G or T and n is an integer ranging from about 5 to about 40, usually from about 8 to about 15, and each member of the population has a random sequence. Typically, the first population described the formula Kₙ includes a plurality of nucleic acid sequences - each made of the same nucleotides, but which have unique random sequences and the second population described the formula Mₙ includes a plurality of nucleic acid sequences - each made of the same nucleotides, but which have unique random sequences.

For example, Kₙ may represent a nucleic acid sequence made-up of a single nucleotide such that it may represent an unlabeled nucleic acid sequence made up of A or C or G or T, and having a size as described above. In yet other embodiments, Kₙ may represent a nucleic acid sequence made-up of two nucleotides such that K is G or T, or K is C or A, or K is C or T, or K is A or T, or K is G or A, or K is G or C, and having a size as described above. In other embodiments, Kₙ may represent an unlabeled nucleic acid sequence made-up of three nucleotides such that K is A or C or G, or K is A or C or T, or K is C or G or T, or K is G or T or A, and having a size as described above. In yet certain other embodiments, Kₙ may represent a nucleic acid sequence made-up of all four nucleotides ATCG. The amount of Kₙ in a sample may vary, but typically an unlabeled nucleic acid having a size from about 5 mers to about 40 mers will be present in a sample in an amount ranging from about 0.001 mg/ml to about 1 mg/ml, usually from about 0.001 mg/ml to about 0.1 mg/ml and more usually from about 0.01 mg/ml to about 0.1 mg/ml. The ratio of unlabeled nucleic acid to labeled nucleic acid may range from about 0.1 to about 10⁵, usually from about 1 to about 10⁵ and more usually from about 10² to about 10⁵.

As described above, in such embodiments, nucleic acids described by the formula Mₙ differ from those nucleic acids described by the formula Kₙ in that at least one different nucleotide is used to make up the nucleic acid represented by Mₙ than is used to make up the nucleic acid represented by Kₙ. For example, in many embodiments all of the nucleotides are different such that K+M=A, C, T G such that all four bases are employed, however the same nucleotide is not present in both K and M.

Accordingly, Mₙ may represent a nucleic acid sequence made-up of a single nucleotide such that M is A or C or G or T having a size as described above. In yet other embodiments, Mₙ may represent a nucleic acid sequence made-up of two nucleotides such that M is G or T, or M is C or A, or M is C or T, or M is A or T, or M is G or A, or M is G or C, and having a size as described above. In other embodiments, Mₙ may represent an unlabeled nucleic acid sequence made-up of three nucleotides such that M is A or C or G, or M is A or C or T, or M is C or G or T, or M is G or T or A, and having a size as described above. In yet certain other embodiments, Mₙ may represent a nucleic acid sequence made-up of all four nucleotides ATCG. The amount of Mₙ in a sample may vary, but typically an unlabeled nucleic acid having a size from about 5 mers to about 40 mers will be present in a sample in an amount ranging from about 0.001 mg/ml to about 1 mg/ml, usually from about 0.001 mg/ml to about 0.1 mg/ml and more usually from about 0.01 mg/ml to about 0.1 mg/ml, where the amount of Mₙ may be the same as Kₘ or may be different. Where there is a difference in the relative amounts of each unlabeled nucleic acid present in a sample, the difference generally does not exceed about 10³ fold and usually does not exceed about 10² fold. The ratio of unlabeled nucleic acid to labeled nucleic acid may range from about 0.1 to about 10⁵, usually from about 1 to about 10⁵ and more usually from about 10² to about 10⁵.

As described above, in certain embodiments, the sample includes at least two different unlabeled nucleic acids, where the different unlabeled nucleic acids differ at least in that they are made up of two different nucleotides and each nucleic acid has a unique random sequence. In one embodiment, Kₙ represents an unlabeled nucleic acid sequence where K is G or T and Mₙ represents a different unlabeled nucleic acid sequence where M is C or A. In other embodiments, Kₙ represents an unlabeled nucleic acid sequence where K is G or A and Mₙ represents an unlabeled nucleic acid sequence where M is C or T, or Kₙ may represents an unlabeled nucleic acid sequence where K is A or T and Mₙ may represents an unlabeled nucleic acid sequence where M is G or A, or Kₙ may represents an unlabeled nucleic acid sequence where K is of G or C and Mₙ may represents an unlabeled nucleic acid sequence where M is A or T. The lengths of these unlabeled nucleic acid sequences made up of two different nucleotides and having a unique random sequence may vary, i.e., may not be the same and/or the unlabeled nucleic acids may be present in substantially proportionate or disproportionate amounts in a sample, as described above.

As mentioned above, the sample suspected of including a nucleic acid analyte and which includes at least a first unlabeled nucleic acid having a random sequence, as described above, e.g., a first nucleic acid made up of at least two types of nucleotides and having a random sequence, is contacted with a nucleic acid array under conditions sufficient to promote binding of complementary sequences to produce binding complexes on the array substrate surface. That is, any sequences in the sample that are complementary to substrate bound sequences will bind therewith to form a binding complex made up of the bound nucleic acid and its complementary nucleic acid in the sample.

As described above, in certain manufacturing protocols employing an array fabricating apparatus, e.g., utilizing pulse jet print technology to synthesize the nucleic acids of the arrays *in situ,* one or more unintended or intermediate (i.e., incomplete) nucleic acid sequences may be synthesized on the substrate surface along with the intended full length nucleic acid sequence(s). This may be caused by the misalignment of the XY stage upon which the array substrate is positioned and/or the misalignment of the alignment system and/or the misalignment of the pulse jet printheads (e.g., the nozzles of the printheads) relative to each other. These unintended sequences may result in binding of labeled target molecules present in the sample to these unintended sequences producing unwanted binding events, as described above, which may compromise the array assay results.

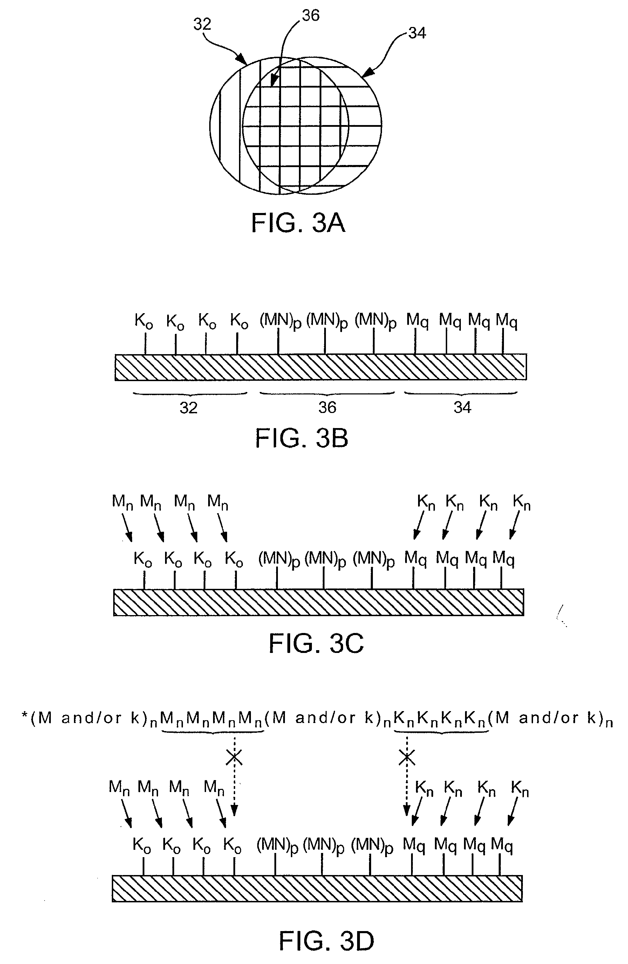

Figures 3A - 3D illustrate how the subject methods prevent the unintended sequences from participating in binding events with labeled analyte present in a sample, if present in a sample that is contacted thereto. Accordingly, Figure 3A schematically illustrates a feature of an array where areas 32 and 34 are present having unintended sequences, as well as area 36 which has the intended full length sequence. These unintended or incomplete sequences may be made of any nucleotide(s) and have any length and/or sequence, e.g., may be made of one or more of A, C, G and T or binding mimetics thereof, depending on, for example, the design of the *in situ* array fabricating apparatus employed (such as a pulse jet printer), the make up and/or sequence and/or size of the intended oligonuclotide, etc. For this particular embodiment, area 32 is described by the formula Kₒ, where K is G or T and area 34 is described by the formula M_{q}, where M is A or C. The variables "o" and "q" are integers ranging from about 5 to about 55, usually from about 10 to about 50 and more usually from about 20 to about 40 such that "o" and "q" are typically less than the number of nucleotides of the intended sequence (KM)ₚ, i.e., the sequence represented in area 36, such that o+q=p where "p" is the intended full length. Area 36 thus includes the full length intended sequence and is described by the formula (KM)ₚ. Figure 3B shows a side-view of the feature of Figure 3A.

As described above, a sample is contacted with the array under suitable binding conditions to promote binding of complementary, labeled nucleic acid target molecules in the sample, if present, to the surface bound sequences. As shown in the side-view in Figure 3C, this particular sample contains at least two different unlabeled nucleic acids, where each is made up of only two types of nucleotides and has a random sequence. That is, the sample contains at least a first unlabeled nucleic acid sequence (usually a plurality of nucleic acid sequences -each usually present in multiple copies) represented by the formula Kₙ, where K is G or T at least a second unlabeled nucleic acid sequence (usually a plurality of nucleic acid sequences -each usually present in multiple copies) described by the formula Mₙ, where M is A or C, and "n" is an integer that may or may not equal "o" or "q".

Accordingly, prior to contact with an array, a sample is prepared such that it includes at least a first unlabeled nucleic acid sequence, where in certain embodiments the sample may include more than one unlabeled nucleic acid sequence, i.e., the sample may include two or more different unlabeled nucleic acids sequences, as described above. The sample may additionally include labeled analyte, as mentioned above and which will be described in greater detail below. The sample will typically be derived from a physiological source. The physiological source may be derived from a variety of eukaryotic sources, where physiological sources of interest including sources derived from single-celled organisms such as yeast and multicellular organisms, including plants and animals, particularly mammals, where the physiological sources from multicellular organisms may be derived from particular organs or tissues of the multicellular organism, or from isolated cells derived therefrom.

In preparing the sample, the various components of the reaction mixture may be combined simultaneously or sequentially. In other words, the one or more unlabeled nucleic acids, and any other additional agents as desired, such as, but not limited to: monovalent and divalent cations, *e.g.* KCl, MgCl₂; sulfhydryl reagents, *e.g.* dithiothreitol; and buffering agents, *e.g.* Tris-Cl. Etc, as desired, may be combined or added to the sample at the same or different times. Generally, the above-described components are combined in an aqueous reaction mixture, where the concentration of each type of unlabeled nucleic acid ranges as described above.

The sample is then contacted with a feature of an array and, under appropriate binding conditions, the unlabeled nucleic acid sequences Kₙ and Mₙ bind to their complementary substrate-bound unintended sequences M_{q} and Kₒ, respectively, as shown in Figure 3C. In this manner, the unintended sequences bound to the substrate surface are not available to bind with any labeled analyte that may be present in the sample.

In certain embodiments, the sample also contains labeled analyte complementary to the intended full length oligonucleotide sequence KM synthesized on the substrate surface. A feature of the subject invention is that the unlabeled nucleic acid present in the sample binds to the unintended sequences on the surface of the substrate, thus preventing binding of the labeled target molecules to these sequences, as described above. Accordingly, in the subject methods unlabeled nucleic acid is present in the sample in an amount sufficient to bind to the unintended sequences on the substrate surface instead of the labeled target analyte molecules binding to the unintended sequences. That is, the unlabeled nucleic acid in the sample is present in an amount sufficient to effectively compete with any labeled analyte that may be present in the sample, where the amount of unlabeled nucleic acid that is sufficient to effectively compete with the labeled target molecules will vary depending on a variety of factors such as the particular length of the unlabeled target nucleic acid, the amount of labeled target present in the sample, the temperature at which the array assay is performed, etc. The effective amount of unlabeled nucleic acid sequences present in sample includes the ranges provided above For example, for a hybridization assay performed at about 60 C, a 0.1 mg/ml mixture of about 10 mers random sequences to about 15 mers random sequences may be used.

Figure 3D illustrates how the unlabeled nucleic acid in the sample effectively competes with the labeled analyte in the sample. Accordingly, sample containing (1) labeled nucleic acid analyte described by the formula (M and/or K)ₙ, (i.e., a mixture of Kₙ, Mₙ and KMₙ), (2) unlabeled nucleic acid described by the formula Kₙ and having a random sequence, and (3) unlabeled nucleic acid described by the formula Mₙ and having a random sequence, is contacted with the feature of Figures 3A and 3B. As shown, the unlabeled poly M and the unlabeled poly K in the sample bind with the unintentionally synthesized poly K and poly M sequences bound to the substrate surface, thus preventing any binding of the labeled nucleic acid analyte to these areas. Accordingly, no signal will be detected in the areas having unintended sequences because the sequences that have hybridized to these areas are unlabeled. However, if complementary, the labeled nucleic acid present in the sample may bind to the intended full length sequence 36.

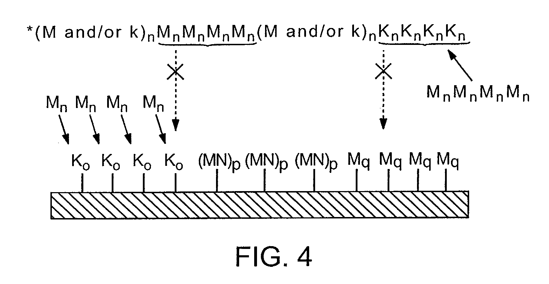

Figure 4 illustrates an alternative embodiment wherein the sample contains labeled nucleic acid analyte complementary to the intended sequences (KM)ₚ and also includes only unlabeled nucleic acid made up of two nucleotides and having a random sequence. In this manner, the unlabeled nucleic acid in the sample will bind to the complementary unintended sequences bound to the surface of the substrate and also to complementary sequences within the labeled nucleic acid analyte. Accordingly, less unlabeled sequences are required to be added to the sample, thus decreasing interactions between the full length intended sequence and the unlabeled nucleic acids and any interaction between the unlabeled nucleic acid sequences is avoided. As illustrated, in this particular example the sample includes unlabeled nucleic acid oligonucleotides described by the formula Mₙ (random CA), as described above. These unlabeled poly M oligonucleotides in the sample bind with both the random poly K oligonucleotides that have been unintentionally synthesized on the substrate surface and complementary sequences of the analyte in the sample, thus preventing the analyte from binding to the poly M oligonucleotides that have been unintentionally synthesized on the substrate surface. Consequently, no signal will be detected in the areas having bound unintended oligonucleotides.

As noted above, the sample is contacted with the array under conditions sufficient or suitable to form binding complexes on the surface of the substrate by the interaction of the surface-bound oligonucleotides and the complementary molecules in the sample. In the case of hybridization assays, the sample is contacted with the array under stringent hybridization conditions. An example of stringent hybridization conditions is hybridization at 50°C or higher and 0.1×SSC (15 mM sodium chloride/1.5 mM sodium citrate). Another example of stringent hybridization conditions is overnight incubation at 42°C in a solution: 50% formamide, 5 × SSC (150 mM NaCl, 15 mM trisodium citrate), 50 mM sodium phosphate (pH7.6), 5 × Denhardt's solution, 10% dextran sulfate, followed by washing the filters in 0.1 × SSC at about 65°C. Hybridization involving nucleic acids generally takes from about 30 minutes to about 24 hours, but may vary as required. Stringent hybridization conditions are hybridization conditions that are at least as stringent as the above representative conditions, where conditions are considered to be at least as stringent if they are at least about 80% as stringent, typically at least about 90% as stringent as the above specific stringent conditions. Other stringent hybridization conditions are known in the art and may also be employed, as appropriate.

Once the incubation step is complete, the array is washed at least one time to remove any unbound and non-specifically bound sample from the substrate, generally at least two wash cycles are used. Washing agents used in array assays are known in the art and, of course, may vary depending on the particular binding pair used in the particular assay. For example, in those embodiments employing nucleic acid hybridization, washing agents of interest include, but are not limited to, salt solutions such as sodium, sodium phosphate and sodium, sodium chloride and the like as is known in the art, at different concentrations and may include some surfactant as well.

Following the washing procedure, as described above, the array is then interrogated or read so that the presence of the binding complexes is then detected i.e., the label is detected using colorimetric, fluorimetric, chemiluminescent or bioluminescent methods. Reading of the array may be accomplished by illuminating the at least one array and reading the location and intensity of resulting fluorescence (or other signal producing system if employed) at each feature of the array to obtain a result. For example, a scanner may be used for this purpose which is similar to the MICROARRAY scanner available from Agilent Technologies, Palo Alto, CA. However, arrays may be read by any other method or apparatus than the foregoing, with other reading methods including other optical techniques (for example, detecting chemiluminescent or electroluminescent labels) or electrical techniques (where each feature is provided with an electrode to detect hybridization at that feature in a manner disclosed in U.S. Patent Nos. 6,251,685; 6,221,583, the disclosure of which is herein incorporated by reference, and elsewhere).

Results from the reading may be raw results (such as fluorescence intensity readings for each feature in one or more color channels) or may be processed results such as obtained by rejecting a reading for a feature which is below a predetermined threshold and/or forming conclusions based on the pattern read from the array (such as whether or not a particular target sequence may have been present in the sample or whether or not a pattern indicates a particular condition of an organism from which the sample came). The results of the reading (whether further processed or not) may be forwarded (such as by communication) to a remote location if desired, and received there for further use (such as further processing).

In certain embodiments, the subject methods include a step of transmitting data from at least one of the detecting and deriving steps, as described above, to a remote location. By "remote location" it is meant a location other than the location at which the array is present and the array assay, e.g., hybridization, occurs. For example, a remote location could be another location (e.g. office, lab, etc.) in the same city, another location in a different city, another location in a different state, another location in a different country, etc. As such, when one item is indicated as being "remote" from another, what is meant is that the two items are at least in different buildings, and may be at least one mile, ten miles, or at least one hundred miles apart. "Communicating" information means transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data. The data may be transmitted to the remote location for further evaluation and/or use. Any convenient telecommunications means may be employed for transmitting the data, e.g., facsimile, modem, internet, etc.

### KITS

Finally, kits for use in array assays are provided. The subject kits include at least a first unlabeled nucleic acid made up of at least two types of nucleotides as described above and instructions for preparing a sample to perform the subject methods. The instructions may be present in the kits as a package insert, in the labeling of the container of the kit or components thereof (i.e., associated with the packaging or sub-packaging) etc. In other embodiments, the instructions are present as an electronic storage data file present on a suitable computer readable storage medium, e.g., CD-ROM, diskette, etc.

The kits may further include a nucleic acid array, e.g., a nucleic acid array having unintended oligonucleotides synthesized thereon. The subject kits may include additional components necessary for carrying out an array assay, such as sample preparation reagents, buffers, labels, and the like. As such, the kits may include one or more containers such as vials or bottles, with each container containing a separate component for the assay, and reagents for carrying out an array assay such as a nucleic acid hybridization assay or the like. The kits may also include a denaturation reagent for denaturing the analyte, buffers such as hybridization buffers, wash mediums, enzyme substrates, reagents for generating a labeled target sample such as a labeled target nucleic acid sample, and negative and positive controls.

### EXPERIMENTAL

The following example is put forth so as to provide those of ordinary skill in the art with a complete disclosure and description of how to make and use the present invention, and are not intended to limit the scope of what the inventors regard as their invention. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

Oligonucleotide arrays were synthesized using a pulse-jet fluid deposition device having two printheads and two phosphoramidite reservoirs per printhead. In this particular example, G and T phosphoramidites were used as coupling reagents in one printhead, while C and A phosphoramidites were used as coupling reagents in the second printhead. All synthesized arrays contained 25,760 features in a 1027 mm2 area and were prepared in a manner analogous to that described above for standard phosphoramidite chemistry on a flat substrate, where the phosphoramidite coupling was controlled spatially by deposition of the appropriate phosphoramidite reagent using the printheads. The oxidation, deblock and, optional capping reactions were performed in flowcells in a non spatially controlled manner.

The oligonucleotide sequences synthesized were 60mer probes complementary to the intronic regions of human genes, and each probe was repeated four times on the array. After synthesis and standard post treatment (deprotection of the base protecting groups, washes, etc.), the arrays were hybridized with a mixture of 1 µg/mL each of Cy3 labeled Hela and Cy5 labeled K562 human samples amplified, labeled and fragmented using Agilent Technologies labeling kit (Agilent model no. G2554A). The hybridization parameters were 17 hours at 60°C. The slides were then washed and dried according to the protocols suggested in the kit, and scanned using an Agilent microarray scanner.

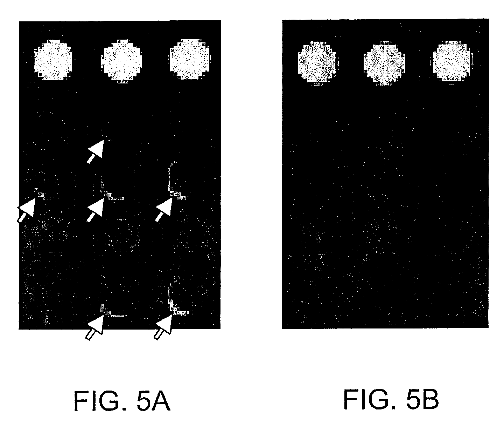

The results showed that when arrays were hybridized without any unlabeled DNA added, crescent- shaped areas from hybridization of the target sample to these unintended were observed as shown in Figure 5A. The arrows shown in Figure 5A show the position of the crescent areas from hybridization of target to unintended sequences. These unintentionally synthesized sequences were due to misalignments in the positions of the printheads during the synthesis. When arrays were hybridized with 0.1 mg/mL of (CA)₁₅ and (GT)₁₅, no crescent-shaped areas were observed as shown in Figure 5B. Data analysis showed that the biological performance of the array (specificity, sensitivity, system noise, etc.) was similar with and without unlabeled DNA added to the hybridization mix thus demonstrating the use of the subject methods, i.e., the addition of unlabeled nucleic acid(s) to the sample, does not adversely affect the array.

It is evident from the above results and discussion that the above described invention provides methods for detecting the presence of a nucleic acid analyte in a sample. The above described invention provides for a number of advantages including ease of use and effective elimination of the results of pulse-jet fluid deposition misalignments, all of which is accomplished without adding additional steps to a conventional analyte detection procotol. As such, the subject invention represents a significant contribution to the art.

All publications and patents cited in this specification are herein incorporated by reference as if each individual publication or patent were specifically and individually indicated to be incorporated by reference. The citation of any publication is for its disclosure prior to the filing date and should not be construed as an admission that the present invention is not entitled to antedate such publication by virtue of prior invention.

While the present invention has been described with reference to the specific embodiments thereof, it should be understood by those skilled in the art that various changes may be made and equivalents may be substituted without departing from the true scope of the invention. In addition, many modifications may be made to adapt a particular situation, material, composition of matter, process, process step or steps, to the objective, and scope of the present invention. All such modifications are intended to be within the scope of the claims appended hereto.

The disclosures in United States patent application no. 10/394,112, from which this application claims priority, and in the abstract accompanying this application are incorporated herein by reference.

## Claims

1. A method of detecting the presence of a nucleic acid analyte in a sample, said method comprising:
(a) contacting a sample suspected of comprising said analyte with a nucleic acid array, wherein said sample comprises at least a first unlabeled nucleic acid made up of at least two types of nucleotide and having a random nucleotide sequence; and
(b) detecting any binding complexes on the surface of said array to obtain binding complex data.

2. A method as claimed in Claim 1, wherein fewer than four types of nucleotide make up said first unlabeled nucleic acid.

3. A method as claimed in Claim 1 or 2, wherein the types of nucleotide that make up said first unlabeled nucleic acid are only two or three of A, C, G and T nucleotides.

4. A method as claimed in Claim 3, wherein the types of nucleotide that make up said first unlabeled nucleic acid are T and G.

5. A method as claimed in Claim 3, wherein the types of nucleotide that make up said first unlabeled nucleic acid are A and C.

6. A method as claimed in any of Claims 1 to 5, wherein said sample comprises a population of two or more different unlabeled nucleic acids, where each member of the population is made up of only the same types of nucleotide and each member of said population has a unique random sequence.

7. A method comprising transmitting data from a first location to a second location, which data comprises a result from a method as claimed in any of claims 1 to 6.

8. A method comprising receiving data transmitted by a method as claimed in Claim 7.

9. A method of determining an unlabeled nucleic acid which should be used with an *in situ* synthesized nucleic acid array to preferentially bind incomplete sequences adjacent array features, the method comprising:
determining a misalignment between different nucleosides deposited onto a surface by an array fabricating apparatus;
determining, as the unlabeled nucleic acid, at least a first unlabeled nucleic acid made up of nucleotides which are selected based on the nucleosides determined to be misaligned, and having a random nucleotide sequence.

10. A kit for use in an array assay, said kit comprising:
(a) at least a first unlabeled nucleic acid made up of two or three types of nucleotide; and
(b) instructions for preparing a sample to perform a method as claimed in any of Claims 1 to 8.
